# EUROPEAN PATENT APPLICATION

(11) **EP 4 246 526 A1**
(43) Date of publication of application: **20.09.2023**
(21) Application number: 22290014.4
(22) Date of filing: 17.03.2022
(51) Int. Cl.: G16H 40/63

(54) **SYSTEM AND METHOD FOR PROVIDING ENHANCING OR CONTRAST AGENT ADVISABILITY INDICATOR**

(71) Applicant: Koninklijke Philips N.V., 5656 AG Eindhoven (NL)
(72) Inventor: Liu, Jin, 5656 AE Eindhoven (NL); Oliveira, Lucas de Melo, 5656 AE Eindhoven (NL); Gessert, Nils Thorben, 5656 AE Eindhoven (NL); Van Den Ham, René, 5656 AE Eindhoven (NL); Waechter-Stehle, Irina, 5656 AE Eindhoven (NL); Wehle, Simon, 5656 AE Eindhoven (NL); De Craene, Mathieu, 5656 AE Eindhoven (NL); Olivier, Antoine, 5656 AE Eindhoven (NL); Prabhu, David, 5656 AE Eindhoven (NL); Eslami, Parastou, 5656 AE Eindhoven (NL); Sun, Deyu, 5656 AE Eindhoven (NL)
(74) Representative: Philips Intellectual Property & Standards

(57) **Abstract**

A system and method for providing an enhancing or contrast agent advisability indicator. Subject data for a subject is processed using an AI-based model to obtain an indication of whether a medical imaging procedure for the subject requires an enhancing or contrast agent. The enhancing or contrast agent advisability indicator is generated with respect to one or more target pathologies assessed in the medical imaging procedure.

## Description

### FIELD OF THE INVENTION

The invention relates to the field of medical imaging, and in particular to medical imaging procedures for which enhancing or contrast agents may be administered.

### BACKGROUND OF THE INVENTION

Enhancing or contrast agents are used in medical imaging procedures to improve visualization of anatomical features and pathologies. In ultrasound procedures, these agents are now generally referred to as "ultrasound enhancing agents" (or "UEAs"), while, for other imaging modalities, the term "contrast agent" is used.

Whether or not an enhancing or contrast agent is used in a medical imaging procedure can affect the accuracy and/or outcome of decisions made based on the images obtained in the procedure. The use of enhancing or contrast agents can have a significant impact on the clinical care of a subject, reducing the need for additional diagnostic procedures and allowing necessary alternations to medical management (e.g. drug regimens) to be identified quickly. However, administration of an enhancing or contrast agent is not without risk to the patient, as it introduces foreign material.

Various clinical guidelines provide guidance as to when an enhancing/contrast agent should be administered, but do not provide objective criteria for determining this. For example, the 2018 American Society of Echocardiography Guidelines Update (Porter, Thomas R. et al., J Am Soc Echocardiogr. 2018, 31(3):241-274) recommends the use of UEAs for all "difficult-to-image" hospitalized patients, but does not set out how to identify "difficult-to-image" cases.

The decision to use an enhancing/contrast agent in a particular procedure is generally taken by the clinician acquiring the images (e.g. a sonographer in the case of ultrasound imaging procedures). However, acquirers of medical images do not usually have expertise in interpreting the images (e.g. for diagnostic purposes) and are therefore not always able to correctly identify when an enhancing/contrast agent is needed or desired to obtain images with sufficient quality for accurate assessment.

In addition to this, clinicians are often under pressure to complete medical imaging procedures quickly. Clinicians can waste time trying to find a suitable position for imaging before realizing that no position will provide an image with sufficient quality unless an enhancing/contrast agent is administered. In some cases, the images obtained in medical imaging procedures completed without an enhancing/contrast agent are later found not to be suitable, and another procedure is required. This creates additional costs, causes patient dissatisfaction and delays treatment.

There is therefore a need for an improved method for determining when to administer an enhancing or contrast agent in a medical imaging procedure.

### SUMMARY OF THE INVENTION

The invention is defined by the claims.

According to examples in accordance with an aspect of the invention, there is provided a processing system for providing an enhancing or contrast agent advisability indicator representative of an advisability of the use of an enhancing or contrast agent in a medical imaging procedure for a subject.

The processing system is configured to: obtain, at an input interface, subject data for the subject; define one or more target pathologies, wherein a target pathology is a pathology that is assessed in the medical imaging procedure; process the subject data using one or more machine-learning algorithms to determine a pathology confidence measure for each of the one or more target pathologies, wherein the value of each pathology confidence measure is representative of a suitability of the medical imaging procedure for assessing the respective target pathology if no enhancing or contrast agent is administered; process the one or more pathology confidence measures to generate an enhancing or contrast agent advisability indicator; and output, at an output interface, the enhancing or contrast agent advisability indicator.

The inventors have recognized that whether or not an enhancing or contrast agent should be administered depends on whether an image obtained during the medical imaging procedure without the use of an enhancing or contrast agent would be suitable for the purpose for which the procedure is being carried out.

For example, if the medical imaging procedure is an echocardiogram for assessing left ventricular function, and an image taken without having administered an enhancing or contrast agent would not enable a clinician to diagnose diseased left ventricular function with sufficient certainty, then an enhancing or contrast agent should be used.

Providing an automatic suggestion of whether or not to administer an enhancing or contrast agent in a medical imaging procedure improves an efficiency of medical imaging, by reducing the time spent determining whether to use an enhancing or contrast agent, reduces a cost of healthcare, by reducing the need for additional diagnostic procedures, and improves an accuracy and reliability of clinical diagnosis.

In some examples, the processing system is configured to generate the enhancing or contrast agent advisability indicator by: defining a pathology confidence threshold; and in response to a determination that at least one of the one or more pathology confidence measures is below the pathology confidence threshold, controlling the enhancing or contrast agent advisability indicator to indicate a recommendation to use an enhancing or contrast agent in the medical imaging process; and in response to a determination that none of the one or more pathology confidence measures is below the pathology confidence threshold, controlling the enhancing or contrast agent advisability indicator to indicate a recommendation to avoid the use of an enhancing or contrast agent in the medical imaging process.

In other words, the processing system recommends the use of an enhancing or contrast agent if any of the one or more pathology confidence measures indicate that images obtained during the medical imaging procedure could not be used to diagnose the respective target pathology with sufficiently high confidence.

If all of the pathology confidence measures indicate that images obtained during the medical imaging procedure could be used to diagnose the respective pathology with sufficiently high confidence, the processing system recommends not using an enhancing or contrast agent.

In some examples, the subject data comprises one or more images acquired during the medical imaging procedure.

In other words, one or more initial images may be acquired during the procedure and analyzed in order to determine whether an assessment of the one or more target pathologies could be made with sufficient certainty based on the initial image(s). A machine-learning algorithm will be able to determine this with greater accuracy than a clinician who is not trained to interpret medical images and diagnose pathologies from them.

In some examples, the subject data comprises at least one of: subject clinical history data, previous imaging data for the subject, subject characteristics and/or pre-procedure data.

Previous imaging data is imaging data obtained before the medical imaging procedure for which the enhancing or contrast agent advisability indicator is generated, e.g., in an earlier imaging session. These types of data are available before the start of a medical imaging procedure, so could be used to generate an enhancing or contrast agent advisability indicator prior to carrying out the imaging procedure. Additionally or alternatively, they may be used alongside one or more images obtained during the medical imaging procedure in order to generate an enhancing or contrast agent advisability indicator during the procedure.

Pre-procedure data may include, for example, a reason for the medical imaging procedure, the type of procedure, subject symptoms and/or an initial diagnosis.

In some examples, the processing system is further configured to receive, from an input user interface, a user input indicative of the one or more target pathologies; and the processing system is configured to define the one or more target pathologies in response to the received user input.

In this way, the clinician carrying out the medical imaging procedure may select the one or more target pathologies that are being assessed in the procedure.

In some examples, the processing system is configured to define the one or more target pathologies by processing the subject data..

This recognizes that likely target pathologies can be determined from the subject data used to determine the pathology confidence measure(s). For example, if pre-procedure data indicates that the reason for the procedure is to assess left ventricular function or that the subject exhibits symptoms of diseased left ventricular function, the processing system would define left ventricular function as a target pathology.

For some types of patient data, natural language processing may be used to process the data in order to define the one or more target pathologies.

In some examples, the one or more machine-learning algorithms have each been trained for a respective pathology, wherein each of the one or more machine-learning algorithms has been trained using a training algorithm configured to receive an array of training inputs and known outputs, wherein the training inputs comprise subject data and the known outputs comprise a determination of whether the respective pathology is present; and the processing system is further configured to select, from a plurality of machine-learning algorithms each trained for a respective pathology, the one or more machine-learning algorithms used to process the subject data in response to the defined one or more target pathologies.

In other words, a plurality of machine-learning algorithms may be trained to determine a pathology confidence measure, with each algorithm trained for a different pathology. The processing system then determines the pathology confidence measure(s) using the machine-learning algorithm(s) corresponding to the one or more target pathologies.

In some examples, the one or more machine-learning algorithms comprise a machine-learning algorithm that has been trained using a training algorithm configured to receive an array of training inputs and known outputs, wherein the training inputs comprise subject data and the known outputs comprise a determination of which of a plurality of pathologies is present.

In other words, a single machine-learning algorithm may be trained to determine a pathology confidence measure for any of a plurality of pathologies.

In some examples, the processing system is further configured to process the subject data using an image quality model to generate a predicted image quality indicator that indicates a predicted image quality of images obtained during the medical imaging procedure if no enhancing or contrast agent is administered; and the processing system is configured to generate the enhancing or contrast agent advisability indicator based on the predicted image quality indicator.

The image quality model may be a statistical model or an AI-based model.

The enhancing or contrast agent advisability indicator may, for example, suggest the use of an enhancing or contrast agent if the predicted image quality indicator indicates that the image quality is predicted to be low if no enhancing or contrast agent is administered.

In some examples, the predicted image quality indicator may also be output at the output interface, to aid clinical decision-making.

In some examples, the processing system is further configured to receive, from an input user interface, a user input indicative of a desired false positive rate; and the processing system is configured to generate the enhancing or contrast agent advisability indicator further based on the input desired false positive rate.

This allows clinicians to choose the cut-off at which use of an enhancing or contrast agent is recommended. This ensures that the advice provided by the processing system is consistent with the clinician's workflow and cost-effectiveness target.

In some examples, the processing system is configured to generate the enhancing or contrast agent advisability indicator further based on a predetermined set of clinical guidance rules.

In other words, when generating the enhancing or contrast agent advisability indicator, the processing system may take into account any relevant clinical guidelines relating to the use of enhancing or contrast agents, such as the American Society of Echocardiography Guidelines.

In some examples, the processing system is configured to: determine a measure of accuracy of the enhancing or contrast agent advisability indicator; and, in response to a determination that the measure of accuracy is below a predetermined accuracy threshold: generate an indication that expert confirmation is required; and output, at the output interface, the indication that expert confirmation is required.

This allows "borderline" cases to be assessed by a clinical expert (e.g. a clinician with experience of interpreting medical images and diagnosing the one or more target pathologies). The request for expert confirmation may be sent directly to the clinical expert (rather than to the clinician acquiring the medical images) for improved efficiency.

If the measure of accuracy is above the predetermined threshold, the processing system may output the enhancing or contrast agent advisability indicator without expert confirmation.

In some examples, the processing system is configured to: receive clinician expertise data representative of training and/or expertise of a clinician acquiring images during the medical imaging procedure; process the clinician expertise data to determine whether the clinician is trained to administer an enhancing or contrast agent; and, in response to a determination that the clinician is not trained to administer an enhancing or contrast agent and that the enhancing or contrast agent advisability indicator recommends use of enhancing or contrast agent: generate an indication that a trained healthcare professional is required to administer an enhancing or contrast agent; and output, at the output interface, the indication that a trained healthcare professional is required to administer an enhancing or contrast agent.

This allows a healthcare professional who is able to administer enhancing or contrast agents to be contacted automatically in the case that use of an enhancing or contrast agent is recommended and the clinician acquiring the medical images is not able to administer the enhancing or contrast agent themselves, further improving the efficiency of the clinical workflow.

If the clinician acquiring the images is determined to be trained to administer an enhancing or contrast agent, the processing system may output the enhancing or contrast agent advisability indicator without also outputting an indication that a trained healthcare professional is required to administer the enhancing or contrast agent.

According to another aspect of the invention, there is provided a computer-implemented method for providing an enhancing or contrast agent advisability indicator representative of an advisability of the use of an enhancing or contrast agent in a medical imaging procedure for a subject.

The computer-implemented method comprises: obtaining, at an input interface, subject data for the subject; defining one or more target pathologies, wherein a target pathology is a pathology that is assessed in the medical imaging procedure; processing the subject data using one or more machine-learning algorithms to determine a pathology confidence measure for each of the one or more target pathologies, wherein the value of each pathology confidence measure is representative of a suitability of the medical imaging procedure for assessing the respective target pathology if no enhancing or contrast agent is administered; processing the one or more pathology confidence measures to generate an enhancing or contrast agent advisability indicator; and outputting, at an output interface, the enhancing or contrast agent advisability indicator.

There is also proposed a computer program product comprising computer program code means which, when executed on a computer device having a processing system, cause the processing system to perform all of the steps of the method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system for providing an enhancing or contrast agent advisability indicator;
Fig. 2 illustrates an example enhancing agent advisability indicator; and
Fig. 3 illustrates a computer-implemented method for providing an enhancing or contrast agent advisability indicator.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

According to a concept of the invention, there is proposed a system and method for providing an enhancing or contrast agent advisability indicator. Subject data for a subject is processed using an AI-based model to obtain an indication of whether a medical imaging procedure for the subject requires an enhancing or contrast agent. The enhancing or contrast agent advisability indicator is generated with respect to one or more target pathologies assessed in the medical imaging procedure.

Embodiments are at least partly based on the realization that machine-learning algorithms may be used to determine whether or not an image taken during a medical imaging procedure without having administered an enhancing or contrast agent would be suitable for clinical purposes.

Illustrative embodiments may, for example, be employed in medical imaging systems (e.g. in an ultrasound cart) and clinical information management systems (e.g. Philips IntelliSpace Cardiovascular system). In ultrasound embodiments, the enhancing or contrast agent advisability indicator is an indication of whether an ultrasound enhancing agent (UEA) should be administered; in embodiments relating to other imaging modalities, the enhancing or contrast agent advisability indicator may be an indication of whether a contrast agent is required.

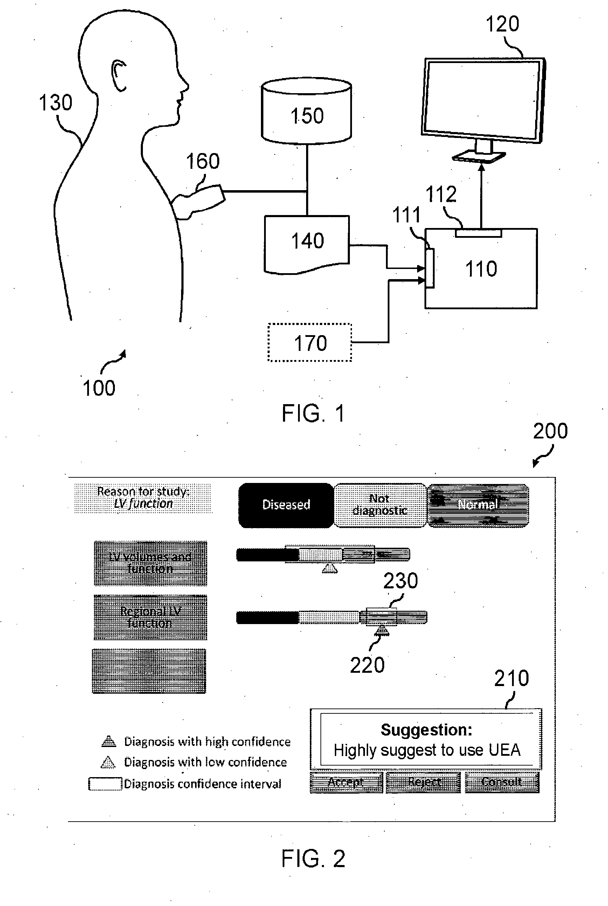

Fig. 1 illustrates a system 100 for providing an enhancing or contrast agent advisability indicator, according to an embodiment of the invention. The system 100 comprises a processing system 110 and an output user interface 120. The processing system is, itself, an embodiment of the invention.

An enhancing or contrast agent advisability indicator is an indicator representative of an advisability of the use of an enhancing or contrast agent in a medical imaging procedure for a subject 130. In Fig. 1, the medical imaging procedure is an echocardiography procedure, but the system 100 may provide any procedure for which an enhancing or contrast agent may be administered. For example, the medical imaging procedure may be an ultrasound imaging procedure, a magnetic resonance imaging (MRI) procedure or a radiography procedure (e.g. a computed tomography (CT) imaging procedure).

When the medical imaging procedure is an ultrasound imaging procedure, as in Fig. 1, the enhancing or contrast agent advisability indicator is an ultrasound enhancing agent (UEA) advisability indicator. For other medical imaging procedures (e.g. MRI or radiography), the enhancing or contrast agent advisability may be a contrast agent advisability indicator. The term "ultrasound enhancing agent", used to describe a class of products comprising microbubbles to enhance ultrasound signals, has replaced the term "ultrasound contrast agent" in order to avoid confusion between such agents and iodinated contrast agents or gadolinium chelates. For other imaging modalities, the term "contrast agent" is still used to refer to agents that are used to enhance medical images.

The processing system 110 obtains, from or at an input interface 111, subject data 140 for the subject 130. In some examples, the subject data comprises one or more images acquired during the medical imaging procedure. Additionally or alternatively, the subject data may comprise data available prior to the medical imaging procedure. For example, the subject data may comprise at least one of: subject clinical history data, previous imaging data for the subject, subject characteristics and/or pre-procedure data.

Subject clinical history data may, for example, include any previous diagnoses for the subject 130 and/or results from previous clinical examinations of the subject. Previous imaging data for the subject may include one or more images from at least one previous medical imaging procedure; the at least one previous medical imaging procedure may include the same type of medical imaging procedure as the procedure for which the enhancing or contrast agent advisability indicator is being provided. Subject characteristics may, for example, include the subject's age, height, weight and/or smoking history. Pre-procedure data is data relating to the medical imaging procedure for which the enhancing or contrast agent advisability indicator is being provided. The pre-procedure data may, for example, include a reason for the medical imaging procedure, the type of procedure, a type of clinician conducting the procedure, subject symptoms and/or an initial diagnosis.

The subject data 140 may be obtained from a memory unit 150. If the subject data comprises one or more images acquired during the medical imaging procedure, the one or more images may be obtained from a medical imaging device 160 used to carry out the medical imaging procedure. Data available prior to the medical imaging procedure may be obtained from any suitable data source, including Electronic Medical Records, Radiology Information System, Cardiology Information System and Picture Archiving and Communication System (PACS). The subject data may be obtained according to an existing IT communication protocol commonly used in healthcare systems, such as Health Level 7 (HL7), Digital Imaging and Communications in Medicine (DICOM) or Fast Healthcare Interoperability Resources (FHIR). In some examples, the subject data may originate from multiple data sources. In some examples, some or all of the subject data may be obtained via user input (e.g. from the clinician carrying out the medical imaging procedure).

In some examples, the subject data 140 may have been pre-processed in order to capture one or more clinical concepts associated with the data. The one or more clinical concepts may be defined using one or more existing ontologies (e.g. SNOMED CT and RadLex). For example, in the case of an echocardiography procedure carried out to assess the subject's left ventricle, clinical concepts may include the subject's age, the subject's height, the subject's weight, the subject's smoking history, "echocardiogram exam", "left ventricle", "contrast", "enlarged left ventricle", "cardiologist" and "ultrasound".

Examples of suitable techniques for capturing clinical concepts associated with subject data include natural language processing (NLP) algorithms that identify clinical concepts in a free-text report, and the use of RegEx expressions to query for clinical concepts in a database. Further suitable techniques will be apparent to the skilled person.

The processing system 110 then defines one or more target pathologies. A target pathology is a pathology that is assessed in the medical imaging procedure. For example, a target pathology for an echocardiogram may be diseased left ventricular function.

In some examples, the processing system defines the one or more target pathologies based on the subject data 140. For example, if the subject data includes pre-procedure data, the one or more target pathologies may be inferred by processing the pre-procedure data. How the processing system processes the subject data to define the one or more target pathologies may depend on the type(s) of data included in the subject data, and suitable methods for defining target pathologies based on various types of data will be apparent to the skilled person. For example, if the subject data includes a text report, the text report may be processed using natural language processing to define the one or more target pathologies. If the subject data has been pre-processed to capture one or more clinical concepts, the one or more clinical concepts may include a target pathology.

In other examples, the system 100 further comprises an input user interface 170, and the processing system 110 receives, from the input user interface (e.g., at the input interface 111), a user input, e.g., a user input signal, indicative of the one or more target pathologies, and defines the one or more target pathologies in response to the received user input. The user input may be provided by the clinician conducting the medical imaging procedure. In some examples, the input user interface may be formed from the output user interface 120 (which may, for example, be a touchscreen device) or a separate device to the output user interface.

Having obtained the subject data 140 and defined the one or more target pathologies, the processing system 110 processes the subject data using one or more machine learning algorithms to determine a pathology confidence measure for each of the one or more target pathologies. The value of each pathology confidence measure is representative of a suitability of the medical imaging procedure for assessing the respective target pathology if no enhancing or contrast agent is administered.

In some examples, each of a plurality of machine-learning algorithms have been trained for a respective pathology, and the processing system 110 selects the one or more algorithms used to process the subject data 140 in response to the defined one or more target pathologies. In other words, the processing system selects, for each target pathology, a particular one or more machine-learning algorithms that have been trained for that pathology.

In other examples, the processing system 110 determines the pathology confidence measure using a single machine-learning algorithm that has been trained for a plurality of pathologies..

A machine-learning algorithm is any self-training algorithm that processes input data in order to produce or predict output data. Here, the input data comprises the subject data 140. If the one or more algorithms have each been trained for a respective pathology, the output data comprises a determination of whether that pathology is present (e.g. a binary yes/no determination or a probability that the pathology is present). If the one or more algorithms is a single algorithm that has been trained for a plurality of pathologies, the output data comprises, for each pathology, a determination of whether the pathology is present.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example subject data. The training output data entries correspond to a determination of whether a particular pathology is present, if the machine-learning algorithm is being trained for a single pathology, or a determination of which (if any) of the plurality of pathologies is present, if the machine-learning algorithm is being trained for a plurality of pathologies.

In some examples, different machine-learning algorithms may be trained for different types (or combinations of types) of subject data. For instance, a first machine-learning algorithm may be trained using input data corresponding to example data that is available prior to a medical imaging procedure, a second machine-learning algorithm may be trained using input data corresponding to images acquired during a medical imaging procedure, and a third machine-learning algorithm may be trained using input data corresponding to both types of subject data. The processing system 110 may then select the machine-learning algorithm(s) used to determine the pathology confidence measure(s) based on the type of subject data 140 for the subject 130. In other examples, each machine-learning algorithm is trained using various types of subject data.

The processing system 110 processes the subject data 140 using the one or more machine-learning algorithms to determine a pathology confidence measure for each of the one or more target pathologies. The pathology confidence measure for each of the one or more target pathologies is a confidence metric (e.g. a confidence level, confidence score or confidence interval) for the decision relating to the target pathology made by the machine-learning algorithm. In other words, the pathology confidence measure is a measure of uncertainty of the determination of whether the target pathology is present.

The pathology confidence measure may, for instance, be a numeric measure (e.g., a score) or a categorical measure.

Methods for measuring an uncertainty of an output of a machine-learning algorithm are well-known, and suitable methods for determining a pathology confidence measure for a target pathology based on an output of a machine-learning algorithm trained to diagnose the target pathology will be readily apparent to the skilled person.

The processing system 110 then processes the one or more pathology measures to generate an enhancing or contrast agent advisability indicator. The enhancing or contrast agent advisability indicator is representative of an advisability of the use of an enhancing or contrast agent in the medical imaging procedure.

The enhancing or contrast agent advisability indicator may be a numeric indicator (e.g., on a predetermined scale such as from 0 to 1, 0 to 10, 1 to 10, 0 to 100 or 1 to 100, etc.), a categorical indicator (e.g., "High", "Medium" or "Low") or a binary indicator (e.g., indicating "Yes" or "No" to advisability of the enhancing or contrast agent).

The enhancing or contrast agent advisability indicator may, for example, be used to control or define a user-perceptible output, such as an audio and/or a visual indicator. For instance, the enhancing or contrast agent advisability indicator may be used to control or define a sound, an image and/or a textual display.

In some examples, the enhancing or contrast agent advisability indicator may simply recommend using an enhancing or contrast agent or recommend using an enhancing or contrast agent. In other examples, the enhancing or contrast agent advisability indicator may also indicate a strength of the recommendation (e.g. "suggest not using UEA", "suggest using UEA", "strongly suggest using UEA", etc.).

The processing system 110 may generate the enhancing or contrast agent advisability indicator based on one or more predetermined rules. For example, if the pathology confidence measure(s) is a numeric value, a pathology confidence threshold may be used to generate the enhancing or contrast agent advisability indicator. The pathology confidence threshold may be defined by the processing system based, for example, on clinical guidelines or on user input.

In some examples, the processing system 110 controls the enhancing or contrast agent advisability indicator to indicate a recommendation use of an enhancing or contrast agent in response to a determination that at least one of the one or more pathology confidence measures is below the pathology confidence threshold. For instance, where each pathology confidence measure is a value on a scale of 0 to 1, the pathology confidence threshold may be a value between 0.3 and 0.7, and preferably a value between 0.4 and 0.6 (e.g. a value of 0.5). If none of the one or more pathology confidence measures is below the pathology confidence threshold, the processing system recommends not using an enhancing or contrast agent.

In some examples, the enhancing or contrast agent advisability indicator may additionally be based on the diagnosis for each target pathology. For example, if the one or more machine-learning algorithms output a probability that a target pathology is present, the processing system 110 may control the enhancing or contrast agent advisability indicator to recommend the use of an enhancing or contrast agent for borderline diagnoses. For instance, a recommendation to use an enhancing or contrast agent may be generated in response to a determination that the probability of a target pathology being present falls within a predefined probability range, such as a range with a lower bound of 20-40% and a higher bound of 60-80% (e.g. a predefined probability range of 30% to 70%).

In some examples, the enhancing or contrast agent advisability indicator may additionally be based on a predicted image quality. For instance, the processing system 110 may process the subject data 140 using an image quality model to generate a predicted image quality indicator. The processing system 110 then uses the predicted image quality indicator to generate the enhancing or contrast agent advisability indicator.

The predicted image quality indicator indicates a predicted image quality of images obtained during the medical imaging procedure if no enhancing or contrast agent is administered. For instance, the predicted image quality indicator may comprise a numerical score (e.g. on a scale of 1 to 5, where 1 represents a lowest image quality and 5 a highest image quality) or a textual indicator (e.g. "high", "medium" or "low").

Suitable image quality models for generating a predicting image quality indicator, including statistical models and AI-based models, will be apparent to the skilled person. If the subject data 140 comprises one or more images acquired during the medical imaging procedure, the image quality model may, for example, be a model that automatically assesses an image quality of the one or more images.

If the subject data 140 additionally or alternatively comprises previous imaging data for the subject, the image quality model may automatically assess an image quality of one or more images generated based on the previous imaging data. It is likely that an enhancing or contrast agent is required if past images that were obtained without using an enhancing or contrast agent have a low image quality. However, images obtained during the current procedure may be considered the best indicator of whether an enhancing or contrast agent is required, and therefore the one or more images acquired during the medical imaging procedure may be prioritized when generating the predicted image quality indicator when such images are available. For instance, more weight may be given to outputs of the image quality model corresponding to images acquired during the medical imaging procedure when generating the predicted image quality indicator.

Models that automatically assess an image quality of a medical image are well-known: see, for example, Amir Abdi et al. (2017), "Quality Assessment of Echocardiographic Cine Using Recurrent Neural Networks: Feasibility on Five Standard View Planes", International Conference on Medical Image Computing and Computer-Assisted Intervention, 302-310, DOI: 10.1007/978-3-319-66179-7_351; and US Patent No. 10,751,029.

For example, the image quality model may be a machine-learning algorithm trained using a retrospectively labeled dataset containing DICOM files labeled according to image quality (e.g. "appropriate", "challenging" and "inappropriate", or a labeled with numerical quality score). Metadata information (e.g. subject information, acquisition timestamps, interpretation timestamps and/or user feedback) may additionally or alternatively be used to train an image quality model.

In some examples, the predicted image quality indicator may be generated by defining an imaging view represented by each of the one or more images acquired during the medical imaging procedure, and assessing the image quality with respect to the defined imaging view. The imaging view may be defined by selecting an imaging view from a plurality of predefined standard views. For example, if the medical imaging procedure is an echocardiogram, the imaging view may be selected from a plurality of predefined standard views including an apical two-chamber view (AP2), an apical three-chamber view (AP3) an apical four-chamber view (AP4).

In other examples, the image quality indicator may be generated based on an assumption that each of the one or more images acquired during the medical imaging procedure could belong to any imaging view. For instance, the predicted image quality indicator could comprise a plurality of quality scores, each assessed according to a different predefined standard view.

In examples in which the enhancing or contrast agent advisability indicator is generated based on one or more predetermined rules, at least one of the one or more predetermined rules may relate to the predicted image quality indicator. For example, the enhancing or contrast agent advisability indicator may recommend the use of an enhancing or contrast agent in response to the predicted image quality indicator predicting a low image quality if no enhancing agent is used. A predetermined image quality threshold may be used to determine whether or not to recommend the use of an enhancing or contrast agent. For instance, if the predicted image quality indicator provides a quality score on a scale of 1 to 5, the enhancing or contrast agent advisability indicator may recommend the use of an enhancing or contrast agent if the score is, for example, below 3, below 2.5 or below 2.

In some examples, the enhancing or contrast agent advisability indicator may be generated further based on a desired false positive rate. The desired false positive rate may be defined by user input, and the processing system 110 may receive a user input indicative of the desired false positive rate from the input user interface 170 and generate the enhancing or contrast agent advisability indicator based on the input desired false positive rate. The processing system may process the desired false positive rate to define a pathology confidence threshold and/or an image quality threshold used to determine the enhancing or contrast agent advisability indicator.

In some examples, the enhancing or contrast agent advisability indicator may be generated further based on a predetermined set of clinical guidance rules. The predetermined set of clinical guidance rules may correspond to published clinical guidelines relating to when to use an enhancing or contrast agent in a medical imaging procedure. Suitable clinical guidelines for determining whether to use an enhancing or contrast agent will be apparent to the skilled person, and will depend on the type of medical imaging procedure being conducted and on where the procedure is taking place. For example, the 2018 American Society of Echocardiography (ASE) Guidelines Update (https://www.asecho.org/wp-content/uploads/2018/03/UEA-Guideline.pdf) would be a suitable set of guidelines if the medical imaging procedure is an echocardiography procedure being carried out in the United States of America.

In some examples, the predetermined set of clinical guidelines may be used to define a pathology confidence threshold and/or an image quality threshold.

In some examples, if the enhancing or contrast agent advisability indicator is generated based on one or more predetermined rules, at least one of the one or more predetermined rules may be predetermined according to clinical guidelines. For instance, the ASE 2018 Guidelines recommends the use of an enhancing or contrast agent when two or more left ventricular segments cannot be visualized adequately. The processing system 110 may therefore generate a recommendation to use an enhancing or contrast agent in response to a determination that it would not be possible to visualize adequately two or more left ventricular segments if no enhancing or contrast agent is used.

Methods for determining whether images obtained during the medical imaging procedure would fulfil the requirements of clinical guidelines will depend on the requirements in question, and suitable methods for particular requirements will be apparent to the skilled person. For example, in the case of a requirement relating to the adequate visualization of left ventricular segments, the processing system 110 may use a segmentation algorithm to attempt to segment the left ventricular wall.

Where a recommendation to use an enhancing or contrast agent is based on clinical guidelines, the enhancing or contrast agent advisability indicator may indicate that this is the case. For example, if the processing system generates a recommendation to use an ultrasound enhancing agent based on the ASE 2018 Guidelines, the enhancing or contrast agent advisability indicator may state "use of UEA recommended based on ASE 2018 Guidelines".

In some examples, the one or more predetermined rules used to generate the enhancing or contrast agent advisability indicator may be applied separately. In other words, if any of the one or more predetermined rules indicates that an enhancing or contrast agent should be used, the processing system 110 will generate an enhancing or contrast agent advisability indicator that recommends the use of an enhancing or contrast agent. For instance, the enhancing or contrast agent advisability indicator may recommend the use of an enhancing or contrast agent if the pathology confidence exceeds the pathology confidence threshold for each target pathology, but the predicted image quality indicator indicates that images obtained during the medical imaging procedure would have a low image quality.

Having generated an enhancing or contrast agent advisability indicator, the processing system 110 outputs the enhancing or contrast agent advisability indicator at the output interface 112.

The enhancing or contrast agent advisability indicator may be provided to an output user interface 120, which may provide a user-perceptible output responsive to the received enhancing or contrast agent advisability indicator (e.g., a visual representation of the enhancing or contrast agent advisability indicator).

The output user interface in Fig. 1 is a display screen; however, the output user interface may be any device suitable for providing a user-perceptible output. The output user interface may, for example, be or comprise a display device, a speaker or a light.

Fig. 2 illustrates an example enhancing agent advisability indicator 200. The example enhancing agent advisability indicator shown in Fig. 2 is an indicator representative of the advisability of the use of a UEA in a cardiac ultrasound procedure. The enhancing agent advisability indicator includes a recommendation to use a UEA 210. In Fig. 2, the enhancing agent advisability indicator also includes supplementary information allowing a clinician to understand why the use of a UEA is recommended. The supplementary information in Fig. 2 includes the diagnosis 220 made by the machine learning algorithm for each target pathology and the confidence interval 230 for each diagnosis (this confidence interval is the pathology confidence measure in the example of Fig. 2). In other examples, supplementary information output alongside a recommendation of whether or not to use an enhancing or contrast agent may include the predicted image quality indicator.

Returning to Fig. 1, in some examples, the system 100 additionally coordinates approval and/or administration of the enhancing or contrast agent.

For example, the processing system 110 may determine a measure of accuracy of the enhancing or contrast agent advisability indicator, and determine whether the measure of accuracy is below a predetermined accuracy threshold. In response to a determination that the measure of accuracy is below the predetermined threshold, the processing system generates an indication that expert confirmation is required and outputs the indication that expert confirmation is required at the output interface 112.

Suitable techniques for determining a measure of accuracy of the enhancing or contrast agent advisability indicator will be apparent to the skilled person. The measure of accuracy may be based on how close the pathology confidence measure is to the pathology confidence threshold. For instance, the processing system 110 may generate an indication that expert confirmation is required if the pathology confidence measure falls within a predetermined range around the pathology confidence threshold (e.g. if the pathology confidence measure is within 10%, 20% or 30% of the pathology confidence measure). The measure or accuracy may additionally or alternatively be based on a level of agreement between the outcomes of the rules and measures used to generate the enhancing or contrast agent advisability indicator. For example, the processing system may generate an indication that expert confirmation is required if the value(s) of the pathology confidence measure(s) do not suggest that an enhancing or contrast agent is required, but the predicted image quality indicator predicts a low image quality if no enhancing or contrast agent is used.

The output interface 112 may pass the indication that expert confirmation is required to an output user interface, which may be the output user interface 120 that outputs the enhancing or contrast agent advisability indicator or a separate device (e.g. a device used by a clinical expert elsewhere in the clinical facility in which the medical imaging procedure is taking place).

The indication that expert confirmation is required may include or be accompanied by information that would enable an expert clinician to determine whether or not an enhancing or contrast agent should be administered in the medical imaging procedure. For instance, the indication that expert confirmation is required may include or be accompanied by some or all of the information comprised in the subject data 140 (e.g. the indication may include one or more images acquired during the medical imaging procedure) and/or the generated enhancing or contrast agent advisability indicator.

If an indication that expert confirmation is required is generated, the processing system 110 may not output the enhancing or contrast agent advisability indicator at the output interface 112 until a user input is received in response to the indication that expert confirmation is required. For example, the processing system may receive, from an input user interface, an indication that an enhancing or contrast agent is required or an indication that an enhancing or contrast agent is not required. The processing system may adjust the enhancing or contrast agent advisability indicator in response to the received user input. For instance, if the processing system has generated an enhancing or contrast agent advisability indicator indicating that the use of an enhancing or contrast agent is not recommended, but a user input from a clinical expert indicates that an enhancing or contrast agent is required, the processing system may adjust the enhancing or contrast agent advisability indicator to recommend the use of an enhancing or contrast agent before the enhancing or contrast agent advisability indicator is output to the output interface 112.

In some examples, the processing system 110 may receive clinician expertise data (not shown in Fig. 1) representative of training and/or expertise of a clinician acquiring images during the medical procedure. The clinician expertise data may, for example, be received by user input or from the memory unit 150. The clinician expertise data may be retrieved from an employee database for the clinical facility at which the clinician is employed.

The processing system 110 may then process the clinician expertise data to determine whether the clinician is trained to administer an enhancing or contrast agent. The processing system may, for example, determine that the clinician is trained to administer an enhancing or contrast agent in response to the clinician expertise data indicating that the clinician has received training in enhancing or contrast agent administration and/or if the clinician expertise data indicates that the clinician has previously administered an enhancing or contrast agent. If the clinician expertise data does not indicate that the clinician has received relevant training or that the clinician has previously administered an enhancing or contrast agent, the processing system may determine that the clinician is not trained to administer an enhancing or contrast agent.

In response to a determination that the clinician is not trained to administer an enhancing or contrast agent, and if the enhancing or contrast agent advisability indicator recommends the use of an enhancing or contrast agent, the processing system 110 may generate an indication that a trained healthcare professional is required to administer an enhancing or contrast agent, and output the indication at the output interface 112.

The output interface 112 may pass the indication that expert confirmation is required to an output user interface, which may be the output interface 120 that outputs the enhancing or contrast agent advisability indicator or a separate device (e.g. a device used by a healthcare professional elsewhere in the clinical facility). In some examples, the processing system 110 may identify a healthcare professional who is trained to administer an enhancing or contrast agent and available to administer an enhancing or contrast agent in the medical imaging procedure, and output the indication that a trained healthcare professional is required to the identified healthcare professional.

In some examples, the processing system 110 may receive a user input, from the input user interface 170, in response to the output enhancing or contrast agent advisability indicator. For example, the user input may indicate that the enhancing or contrast agent advisability indicator is accepted or rejected. In some examples, the processing system may only determine whether to generate an indication that a trained healthcare professional if a recommendation to use an enhancing or contrast agent is accepted by user input. In some examples, the processing system may receive a user input request to consult an expert clinician, and generate an indication that expert confirmation is required in response to receiving the user input request.

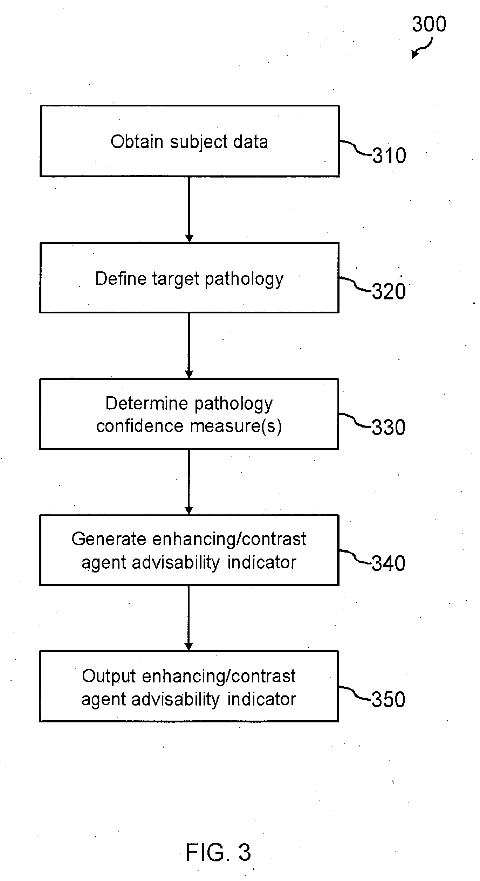

Fig. 3 illustrates a computer-implemented method 300 for providing an enhancing or contrast agent advisability indicator representative of an advisability of the use of an enhancing or contrast agent in a medical imaging procedure for a subject, according to an embodiment of the invention.

The method begins at step 310, at which subject data for the subject is obtained at an input interface.

At step 320, one or more target pathologies are defined. A target pathology is a pathology that is assessed in the medical imaging procedure.

At step 330, the subject data is processed using one or more machine-learning algorithms to determine a pathology confidence measure for each of the one or more target pathologies. The value of each pathology confidence measure is representative of a suitability of the medical imaging procedure for assessing the respective target pathology if no enhancing or contrast agent is administered.

At step 340, the one or more pathology confidence measures are processed to generate an enhancing or contrast agent advisability indicator.

At step 350, the enhancing or contrast agent advisability indicator is output at an output interface.

The method 300 may be carried out before commencing the medical imaging procedure if the subject data comprises only data available prior to the medical imaging procedure. Additionally or alternatively, the method may be carried out during the medical imaging procedure (e.g. at the start of the medical imaging procedure).

It will be understood that the disclosed methods are computer-implemented methods. As such, there is also proposed a concept of a computer program comprising code means for implementing any described method when said program is run on a processing system.

The skilled person would be readily capable of developing a processor for carrying out any herein described method. Thus, each step of a flow chart may represent a different action performed by a processor, and may be performed by a respective module of the processor.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A processing system (110) for providing an enhancing or contrast agent advisability indicator (200) representative of an advisability of the use of an enhancing or contrast agent in a medical imaging procedure for a subject (130), the processing system being configured to:
obtain, at an input interface (111), subject data (140) for the subject;
define one or more target pathologies, wherein a target pathology is a pathology that is assessed in the medical imaging procedure;
process the subject data using one or more machine-learning algorithms to determine a pathology confidence measure for each of the one or more target pathologies, wherein the value of each pathology confidence measure is representative of a suitability of the medical imaging procedure for assessing the respective target pathology if no enhancing or contrast agent is administered;
process the one or more pathology confidence measures to generate an enhancing or contrast agent advisability indicator; and
output, at an output interface (112), the enhancing or contrast agent advisability indicator.

2. The processing system (110) of claim 1, wherein the processing system is configured to generate the enhancing or contrast agent advisability indicator (200) by:
defining a pathology confidence threshold;
in response to a determination that at least one of the one or more pathology confidence measures is below the pathology confidence threshold, controlling the enhancing or contrast agent advisability indicator to indicate a recommendation to use an enhancing or contrast agent in the medical imaging process; and
in response to a determination that none of the one or more pathology confidence measures is below the pathology confidence threshold, controlling the enhancing or contrast agent advisability indicator to indicate a recommendation to avoid the use of an enhancing or contrast agent in the medical imaging process.

3. The processing system (110) of claim 1 or 2, wherein the subject data (140) comprises one or more images acquired during the medical imaging procedure.

4. The processing system (110) of any of claims 1 to 3, wherein the subject data (140) comprises at least one of: subject clinical history data, previous imaging data for the subject, subject characteristics and/or pre-procedure data.

5. The processing system (110) of any of claims 1 to 4, wherein:
the processing system is further configured to receive, from an input user interface (170), a user input indicative of the one or more target pathologies; and
the processing system is configured to define the one or more target pathologies in response to the received user input.

6. The processing system (110) of any of claims 1 to 4, wherein the processing system is configured to define the one or more target pathologies by processing the subject data (140).

7. The processing system (110) of any of claims 1 to 6, wherein:
the one or more machine-learning algorithms have each been trained for a respective pathology, wherein each of the one or more machine-learning algorithms has been trained using a training algorithm configured to receive an array of training inputs and known outputs, wherein the training inputs comprise subject data and the known outputs comprise a determination of whether the respective pathology is present; and
the processing system is further configured to select, from a plurality of machine-learning algorithms each trained for a respective pathology, the one or more machine-learning algorithms used to process the subject data (140) in response to the defined one or more target pathologies.

8. The processing system (110) of any of claims 1 to 6, wherein the one or more machine-learning algorithms comprise a machine-learning algorithm that has been trained using a training algorithm configured to receive an array of training inputs and known outputs, wherein the training inputs comprise subject data and the known outputs comprise a determination of which of a plurality of pathologies is present.

9. The processing system (110) of any of claims 1 to 8, wherein:
the processing system is further configured to process the subject data (140) using an image quality model to generate a predicted image quality indicator that indicates a predicted image quality of images obtained during the medical imaging procedure if no enhancing or contrast agent is administered; and
the processing system is configured to generate the enhancing or contrast agent advisability indicator (200) based on the predicted image quality indicator.

10. The processing system (110) of any of claims 1 to 9, wherein:
the processing system is further configured to receive, from an input user interface (170), a user input indicative of a desired false positive rate; and
the processing system is configured to generate the enhancing or contrast agent advisability indicator (200) further based on the input desired false positive rate.

11. The processing system (110) of any of claims 1 to 10, wherein the processing system is configured to generate the enhancing or contrast agent advisability indicator (200) further based on a predetermined set of clinical guidance rules.

12. The processing system (110) of any of claims 1 to 11, wherein the processing system is configured to:
determine a measure of accuracy of the enhancing or contrast agent advisability indicator (200); and, in response to a determination that the measure of accuracy is below a predetermined accuracy threshold:
generate an indication that expert confirmation is required; and
output, at the output interface (112), the indication that expert confirmation is required.

13. The processing system (110) of any of claims 1 to 12, wherein the processing system is configured to:
receive clinician expertise data representative of training and/or expertise of a clinician acquiring images during the medical imaging procedure;
process the clinician expertise data to determine whether the clinician is trained to administer an enhancing or contrast agent; and, in response to a determination that the clinician is not trained to administer an enhancing or contrast agent and that the enhancing or contrast agent advisability indicator recommends use of an enhancing or contrast agent:
generate an indication that a trained healthcare professional is required to administer an enhancing or contrast agent; and
output, at the output interface (112), the indication that a trained healthcare professional is required to administer an enhancing or contrast agent.

14. A computer-implemented method (300) for providing an enhancing or contrast agent advisability indicator (200) representative of an advisability of the use of an enhancing or contrast agent in a medical imaging procedure for a subject (130), the computer-implemented method comprising:
obtaining, at an input interface (111), subject data (140) for the subject;
defining one or more target pathologies, wherein a target pathology is a pathology that is assessed in the medical imaging procedure;
processing the subject data using one or more machine-learning algorithms to determine a pathology confidence measure for each of the one or more target pathologies, wherein the value of each pathology confidence measure is representative of a suitability of the medical imaging procedure for assessing the respective target pathology if no enhancing or contrast agent is administered;
processing the one or more pathology confidence measures to generate an enhancing or contrast agent advisability indicator; and
outputting, at an output interface (112), the enhancing or contrast agent advisability indicator.

15. A computer program product comprising computer program code means which, when executed on a computer device having a processing system, cause the processing system to perform all of the steps of the method (300) according to claim 14.
